# EUROPÄISCHE PATENTSCHRIFT

(11) **EP 2 452 614 B1**
(45) Veröffentlichungstag und Bekanntmachung des Hinweises auf die Patenterteilung: **28.09.2016**
(21) Anmeldenummer: 11188447.4
(22) Anmeldetag: 09.11.2011
(51) Int. Cl.: A61B 1/24, A61B 5/00, A61B 1/00, A61B 1/06

(54) **Zahnärztliches System zum Transilluminieren von Zähnen**
Dental system for transillumination of teeth
Système de médecine dentaire destiné à transilluminer des dents

(30) Priorität: 11.11.2010 DE 102010043792
(43) Veröffentlichungstag der Anmeldung: 16.05.2012
(73) Patentinhaber: Kaltenbach & Voigt GmbH, 88400 Biberach (DE)
(72) Erfinder: Hackel, Andre, 88400 Biberach (DE); Heckenberger, Hans, 88433 Aßmannshardt (DE); Erdmann, Sven, 89081 Ulm (DE)
(74) Vertreter: Thun, Clemens

(56) Entgegenhaltungen:
- EP-A1- 1 136 034
- EP-A1- 1 595 494
- WO-A1-2007/067776
- DE-A1-102007 013 355
- US-B1- 6 672 868

## Beschreibung

Die vorliegende Erfindung betrifft ein zahnärztliches System zum Transilluminieren von Zähnen gemäß dem Oberbegriff des Anspruchs 1, welches eine Lichtquelle zum Erzeugen einer Untersuchungsstrahlung, Bestrahlungsmittel zum Richten der Untersuchungsstrahlung auf einen zu untersuchenden Zahn sowie Mittel zum Erfassen eines optischen Bildes des durch die Untersuchungsstrahlung illuminierten Zahns aufweist.

In der Medizin, insbesondere der Zahnmedizin werden vermehrt Diagnosesysteme eingesetzt, welche auf optischen Prinzipien beruhen. Grund hierfür ist, dass derartige Geräte üblicherweise berührungslos, also insbesondere schmerzfrei die Erstellung einer Diagnose ermöglichen und darüber hinaus auch oftmals optische Bilder zur Verfügung stellen, anhand welcher eventuell erforderliche therapeutische Maßnahmen dem Patienten anschaulich und damit besser vermittelt werden können. Beispielsweise kommen in der Zahnmedizin sogenannte Intraoralkameras zum Einsatz, welche ein Handstück beinhalten, dessen vorderer Endbereich in den Mund eines Patienten eingeführt wird. In diesem Endbereich befindet sich in der Regel ein Lichteintritts- bzw. Sichtfenster für die Kameraoptik, von dem ausgehend das Bild des zu untersuchenden Objekts einer Erfassungseinrichtung, beispielsweise einem CCD-Chip übermittelt wird.

Eine derartige Intraoralkamera kann ferner zu einem System zum Transilluminieren von Zähnen erweitert werden, wie es u. a. aus der DE 10 2006 041 020 A1 der Anmelderin bekannt ist. Hierbei wird der zu untersuchende Zahn mit Licht innerhalb eines bestimmten Wellenlängenbereichs bestrahlt, wobei dann ein optisches Bild des durch die Untersuchungsstrahlung illuminierten Zahns erfasst und bewertet wird. Da kariöse Stellen im Zahn das Licht anders streuen als gesundes Zahngewebe, können derartige Stellen bei Beobachtung des Zahns mit Hilfe einer Kamera identifiziert werden, wobei bei entsprechender Ausgestaltung des Systems sogar eine zuverlässigere Kariesdiagnose möglich ist als dies bei einer klassischen Röntgenuntersuchung der Fall ist.

Die Qualität der bei dieser Vorgehensweise erstellten Aufnahmen und damit letztendlich der Kariesdiagnose hängt in entscheidendem Maße davon ab, in welcher Weise das Licht in den zu illuminierenden Zahn eingekoppelt wird. In der DE 10 2006 041 020 A1 wird hierzu ein spezielles Kopfstück eines Instruments verwendet, welches an dem Zahn anliegt. Kommt es hierbei zu Überstrahlungen in Folge von direkter oder durch Reflektion hervorgerufener Belichtung des Lichtsensors, so wird eine rechentechnische Eliminierung dieser Bildbereiche durchgeführt. Es ergibt sich hierbei allerdings der Nachteil, dass aufgrund der rechentechnischen Möglichkeiten der Dynamikumfang des Systems eingeschränkt wird.

Alternativ hierzu ist aus der US 4,184,175 bekannt Polarisationsfilter einzusetzen, um Reflektionslicht oder direktes Licht auszublenden. Durch die Polarisation wird allerdings ein Intensitätsverlust bei der Beleuchtung des Zahns hervorgerufen, was einen nicht unerheblichen Nachteil darstellt. Schließlich beschreibt die US 6, 672,868 eine Vorrichtung, die über mechanische Hilfsmittel zur Lichtblockung verfügt, wodurch der direkte Eingang von Reflektionslicht oder direktem Licht im Lichtsensor vermindert wird. Derartige Maßnahmen führen allerdings zu einer vergrößerten Bauweise des vorderen Endbereichs des Geräts, was auf Grund des ohnehin reduzierten Bewegungsspielraums innerhalb des Mundraums des Patienten nachteilig ist.

Aus der US 6,672,868 B1 ist ein zahnärztliches System zum Transilluminieren von Zähnen bekannt, bei dem das Licht zur Bestrahlung des Zahns mit Hilfe eines Lichtleiters zu Zahnoberfläche gelenkt wird. Der entsprechende Endbereich des Lichtleiters ist von einem elastischen Positionierelement umgeben.

Aus der EP 1 595 494 A1 ist ein zahnärztliches System zum Untersuchen der optischen Eigenschaften von Zahngewebe bekannt. Bei diesem System wird Licht über einen transparenten Lichtkeil auf die Zahnoberfläche gelenkt.

Aus der DE 10 2007 013 355A1 ist eine dentale Diagnosekamera bekannt. Diese weist einen Abstandshalterabschnitt auf, der zur Diagnose auf den zu untersuchenden Zahn aufgesetzt wird.

Der vorliegenden Erfindung liegt dementsprechend die Aufgabe zugrunde, eine neuartige Lösung zur Realisierung eines Systems zum Transilluminieren von Zähnen anzugeben, bei dem die oben genannten Nachteile vermieden werden. Insbesondere soll eine effektive und zuverlässige Einkopplung des Lichts in den zu transilluminierenden Zahns sichergestellt sein.

Die Aufgabe wird durch ein zahnärztliches System zum Transilluminieren von Zähnen, welches die Merkmale des Anspruchs 1 aufweist, gelöst. Vorteilhafte Weiterbildungen der Erfindung sind Gegenstand der abhängigen Ansprüche.

Die erfindungsgemäße Lösung beruht auf dem Gedanken, die Art und Weise, in der Licht in den zu transilluminierenden Zahn eingekoppelt wird, zu optimieren. Hierfür wird gemäß einem ersten Aspekt der vorliegenden Erfindung vorgeschlagen, ein dem zu untersuchenden Zahn zugewandtes lichtleitendes oder lichtlenkendes Element, mit dessen Hilfe das Licht letztendlich auf den Zahn gerichtet wird, in einen transparenten, flexiblen Einkopplungskörper einzubetten, der zur Anlage an den Zahn bzw. das Zahnfleisch des Zahns, die sogenannte Gingiva, vorgesehen ist.

Gemäß diesem ersten Aspekt der vorliegenden Erfindung wird dementsprechend ein zahnärztliches System zum Transilluminieren von Zähnen vorgeschlagen, welches eine Lichtquelle zum Erzeugen einer Untersuchungsstrahlung, Bestrahlungsmittel zum Richten der Untersuchungsstrahlung auf einen zu untersuchenden Zahn sowie Mittel zum Erfassen eines optischen Bildes des durch die Untersuchungsstrahlung illuminierten Zahns aufweist, wobei die Bestrahlungsmittel lichtlenkende und/oder lichtleitende Elemente umfassen und wobei zumindest das dem zu untersuchenden Zahn zugewandte lichtleitende und/oder lichtlenkende Element in einen transparenten, flexiblen Einkopplungskörper eingebettet ist, der zur Anlage an den Zahn bzw. die Gingiva des Zahns vorgesehen ist.

Dadurch, dass die Lichteinkopplung in den Zahn mit Hilfe des flexiblen Einkopplungskörpers vorgenommen wird, wird ein störender Lichtaustritt aus dem Lichtleitelement verhindert oder zumindest reduziert. Es wird ein maximaler Einkopplungsgrad für das Licht in die Zahnsubstanz bzw. Gingiva erhalten, was letztendlich zu verbesserten Aufnahmen des zu untersuchenden Zahns führt.

Die Einkoppelgeometrie des transparenten Einkopplungskörpers besteht aus einer planen Fläche, einer Kugel, einem Kugelsegment, einer konkaven bzw. konvexen Fläche oder aus einer Freiformfläche. Diese Flächen sind dann erfindungsgemäß mit einer Zusatzstruktur ausgestattet, welche verhindert, dass Licht, welches nicht in die Zahnhartsubstanz und/oder Gingiva eingekoppelt werden kann, an dem Zahn und/oder der Gingiva reflektiert wird und zu einer Überblendung des Bildaufnahmesensors oder eines anderen lichtempfindlichen Detektors führt. Die Funktionsweise dieses Prinzips beruht dabei darauf, dass Lichtstrahlen an den Flächen der Zusatzstruktur teilweise umgelenkt werden, solange die entsprechenden Strukturen nicht mit der Zahnhartsubstanz und/oder der Gingiva in Kontakt kommen. Der Effekt beruht dabei auf dem Unterschied im Brechungsindex zwischen dem Kontaktmaterial und Luft, der einen Lichtaustritt aus dem Einkopplungskörper nur unter bestimmten Winkeln zulässt. Sind die Strukturen des Einkopplungskörpers nicht in Anlage gegen den Zahn bzw. die Gingiva, so wird das Licht aufgrund der Verhältnisse vermehrt zurückreflektiert und nicht ausgekoppelt. Geeignet für diese Zusatzstrukturen sind Geometrien in Form von Pyramiden, Kegeln, Wellen sowie Wellen mit einer beliebigen geeigneten Struktur, die periodisch und/oder unperiodisch verteilt ist.

Neben einer Verbesserung der Lichteinkopplung an dem zu untersuchenden Zahn besteht eine weitere Funktion des Einkopplungskörpers darin, die lichtleitenden und/oder lichtlenkenden Elemente, mit deren Hilfe das Licht von der Lichtquelle auf den Zahn gerichtet wird, vor äußeren Einflüssen zu schützen. Dies ist deshalb von Bedeutung, da es aus hygienischen Gründen zwingend erforderlich ist, den in den Mundraum des Patienten eingeführten Bereich des Geräts regelmäßig zu reinigen, insbesondere aber auch zu sterilisieren. Durch das Einbetten der lichtleitenden/lichtlenkenden Elemente werden diese besser geschützt, so dass diese durch den Sterlilisierungsvorgang, bei dem das Gerät hohen Temperaturen sowie einer hohen Luftfeuchtigkeit ausgesetzt ist, nicht beschädigt wird. Dabei kann insbesondere vorgesehen sein, dass Lichtleiter, welche sich von einer Lichtquelle bis zur Lichtauskopplungsstelle erstrecken, in das transparente Material des Einkopplungskörpers eingebettet sind. Am vordersten Ende des Lichtleiters kann sich dann ein Prisma befinden, das letztendlich die Strahlumlenkung bewirkt, um das Licht auf den zu untersuchenden Zahn zu richten. Dabei hat sich gezeigt, dass die Schutzwirkung für die Elemente zur Lichtübertragung zusätzlich verbessert werden kann, wenn eine hydrophobe Beschichtung verwendet wird. Diese könnte beispielsweise direkt auf den Lichtleiter oder auf eine Ummantelung des Lichtleiters aufgebracht werden, um zu verhindern, dass Feuchtigkeit in die darunter liegenden Bauteile eindringen und diese beschädigen kann. Beispielsweise könnte hierfür das Material Parylene verwendet werden.

Eine weitere vorteilhafte Maßnahme der vorliegenden Erfindung dient dazu, das Entstehen sogenannter Speckle, die durch Interferenzerscheinungen im humanen Gewebe hervorgerufen werden, zu vermeiden bzw. zumindest zu verringern. Hierzu wird vorgeschlagen, den zu untersuchenden Zahn mit einer verhältnismäßig geringen spektralen Bandweite im Bereich zwischen 2 und 10nm zu bestrahlen.

Ferner kann erfindungsgemäß eine besondere Anordnung der Lichtquellen innerhalb des Handgeräts vorgesehen sein. Diese besondere Anordnung ist darauf zurückzuführen, dass zur Übertragung des Lichts Lichtleiter verwendet werden, die allerdings aufgrund des erforderlichen Platzes für die Bildübertragung zu den Bilderfassungsmitteln nicht zentral durch das Handstück verlaufen können. In diesem Fall hat sich eine schräge Ausrichtung der Lichtquellen gegenüber der Längsachse des Handstücks als vorteilhaft herausgestellt, da auf diesem Wege eine maximale Transmission des ausgesendeten Lichts erzielt werden kann, wobei gleichzeitig der Biegeradius des Lichtleiters optimiert werden kann, und - wie später ausführlich erläutert wird - hierdurch eine möglichst kleine Bauweise für das Handstück ermöglicht wird.

Schließlich wird gemäß eines weiteren Aspekts der vorliegenden Erfindung ein speziell ausgestalteter Aufsatz zur Durchleuchtung eines zu untersuchenden Zahns vorgeschlagen, bei der von einer Seite her der Zahn beleuchtet und von der gegenüberliegenden Seite her das Bild des illuminierten Zahns erfasst wird. Dadurch, dass der Aufsatz zwar abnehmbar von dem Handstück gehalten wird, allerdings im Vergleich zur Längsachse des Handstücks feststehend angeordnet ist, wird eine wesentliche Arbeitserleichterung und Arbeitszeitersparnis erzielt, da umgehend die Betrachtungsrichtung gewechselt werden kann.

Letztendlich tragen die oben geschilderten Maßnahmen dazu bei, dass einerseits die Qualität der erzielbaren Aufnahmen und damit die Zuverlässigkeit der Kariesdiagnose sowie andererseits die Handhabung des Geräts vereinfacht wird.

Nachfolgend soll die Erfindung anhand der beiliegenden Zeichnung näher erläutert werden. Es zeigen:
- Figur 1: die Ansicht eines ersten Ausführungsbeispiels eines Handgeräts zum Transilluminieren von Zähnen;
- Figur 2: eine vergrößerte Darstellung des vorderen Endbereichs des Handgeräts;
- Figur 2a: schematisch die Einkopplung des Lichts in die Gingiva;
- Figur 3: eine Schnittdarstellung des Geräts von Figur 1;
- Figur 4: eine Darstellung der Anordnung der Lichtquellen innerhalb des Handgeräts;
- Figuren 5-8: verschiedene Ansichten des vorderen Endbereichs des Aufsatzes des Handgeräts mit dem Verlauf der zur Lichteinkopplung genutzten Lichtleiter;
- Figur 9-12: Ansichten des Lichtleiterhalters mit dem transparenten Einkopplungskörper;
- Figur 13: den transparenten Einkopplungskörper mit dem darin befindlichen Umlenkprisma;
- Figuren 14 und 15: Darstellung des Lichtleiters mit dem daran am vorderen Ende befindlichen Umlenkprisma;
- Figur 16: schematisch die Abgabe des Lichts über den Einkopplungskörper;
- Figuren 17-19: verschiedene Ansichten einer an der Oberfläche des Einkopplungskörpers befindlichen Struktur;
- Figur 20: die Funktionsweise der an dem Einkopplungskörper vorgesehenen Struktur;
- Figur 21: eine perspektivische Ansicht eines alternativen Aufsatzes zur Lichteinkopplung und Bilderfassung des erfindungsgemäßen Geräts;
- Figur 22: eine Schnittdarstellung des Aufsatzes von Figur 20 und
- Figuren 23 bis 25: systematische Darstellungen zur Verwendung des Aufsatzes von Figur 21.

Zunächst soll nachfolgend das der vorliegenden Erfindung zugrunde liegende Transilluminations-Verfahren grundsätzlich erläutert werden.

Dieses Transilluminations-Verfahren beruht darauf, einen Zahn mit sichtbarem Licht zu durchleuchten bzw. zu transilluminieren. Hierzu wird mit Hilfe einer Lichtquelle eine Untersuchungsstrahlung generiert, welche auf den Zahn gerichtet wird. Die Untersuchungsstrahlung liegt dabei üblicherweise in einem Wellenlängenbereich von etwa 550 µm bis 790 µm, z. B. bei etwa 670 µm. Das Gewebe des Zahns blockt diese Untersuchungsstrahlung nicht vollständig ab, sondern erlaubt stattdessen, dass das Licht durch den Zahn hindurchtritt. Hierbei wird die Strahlung teilweise gestreut, wobei insbesondere kariöse Bereiche für eine charakteristische Beeinflussung des Lichts sorgen. Wird der auf diese Weise transilluminierte Zahn aus verschiedenen Blickrichtungen betrachtet, so können diese kariösen Bereiche erkannt werden, da diese etwas dunkler erscheinen. Insbesondere dann, wenn Aufnahmen, die zu verschiedenen Zeitpunkten erstellt wurden, miteinander verglichen werden, kann Karies auf diese Weise verhältnismäßig effektiv und auch frühzeitig erkannt werden.

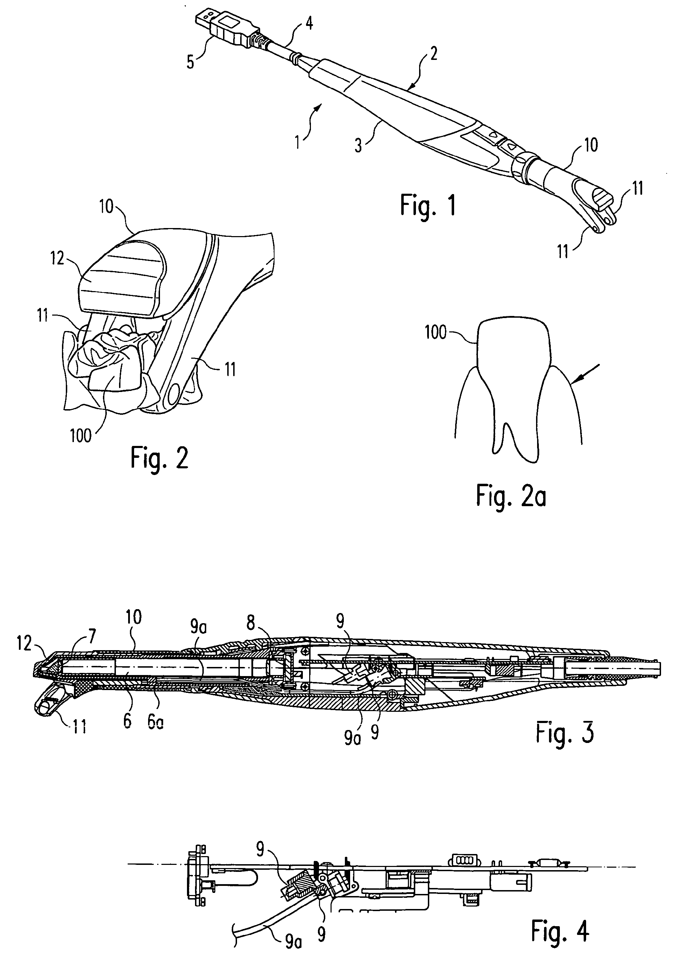

Ein dieses Prinzip nutzendes Gerät ist in Figur 1 dargestellt und allgemein mit dem Bezugszeichen 1 versehen. Es weist als wesentlichen Bestandteil ein längliches Handgerät 2 mit einer Griffhülse 3 auf, in der die wesentlichen Komponenten zur Beleuchtung des zu untersuchenden Zahns sowie zur Bilderfassung angeordnet sind. Insbesondere befinden sich innerhalb der Griffhülse 3 Lichtquellen zum Erzeugen einer Untersuchungsstrahlung sowie Bilderfassungsmittel, beispielsweise in Form eines CCD-Chips zur Aufnahme eines mit Hilfe des Lichts illuminierten Zahns. Die von den Bilderfassungsmitteln erstellten Daten werden dann an eine Zentrale übermittelt. Hierzu erstreckt sich vom hinteren Ende der Griffhülse 3 aus ein Kabel 4, an dessen Ende sich ein USB-Stecker 5 befindet, über den in einfacher Weise ein Anschluss zu einem PC hergestellt werden kann. Auf diesem - nicht dargestellten - PC können dann die Bildinformationen gespeichert und analysiert werden. Anstelle des dargestellten USB-Anschlusses könnte das Handgerät 2 auch auf anderem Wege mit einer Zentrale verbunden werden. Die USB-Verbindung hat sich allerdings als besonders vorteilhaft erwiesen, da hierüber gleichzeitig auch das Gerät 1 mit Strom versorgt werden kann.

Die Anordnung der Lichtquellen sowie der Bilderfassungsmittel innerhalb der Griffhülse 3 des Handstücks 2 bringt zunächst den Vorteil mit sich, dass diese Komponenten hier verhältnismäßig gut von äußeren Einflüssen geschützt sind. Die eigentliche Beleuchtung des Zahns erfolgt dann mit Hilfe eines Aufsatzes 10, der lösbar am vorderen Ende der Griffhülse 3 angeordnet ist. Wie die Schnittdarstellung von Figur 3 zeigt, weist die Griffhülse 3 an ihrem vorderen Ende einen zylinderförmigen Zapfen 6 auf, auf den der Aufsatz 10 aufgesetzt wird. Am vorderen Ende des Aufsatzes 10 sind zwei seitliche Arme 11 ausgebildet, über welche jeweils entsprechend der Darstellung von Figur 2 das Licht auf Seitenflächen eines zu untersuchenden Zahns 100 bzw. auf das Zahnfleisch des Zahns 100, die sogenannte Gingiva gerichtet werden kann. Wie Figur 2a zeigt, erfolgt hierbei die Lichteinkopplung idealerweise leicht schräg im oberen Endbereich der Gingiva, da sich gezeigt hat, dass in diesem Fall der höchste Kontrast für den durchleuchteten Zahn 100 erreicht wird. Die nähere Ausgestaltung der Arme 11 mit den darin befindlichen Elementen zur Lichtübertragung wird später ausführlich erläutert.

Der Lichteintritt zur Beobachtung des illuminierten Zahns erfolgt mit Hilfe eines Fensters, welches an der Unterseite des vorderen Endbereichs 12 des Aufsatzes 10 ausgebildet ist. Der zylinderförmige Zapfen 6 des Handgeräts 2 erstreckt sich bis in diesen vorderen Endbereich 12 des Aufsatzes 10 derart, dass über das Fenster ein Lichteintritt in ein Umlenkprisma 7 ermöglicht wird. Über dieses werden eintretende Lichtstrahlen derart umgelenkt, dass sie auf die in der Griffhülse 3 befindlichen Bilderfassungsmittel, also beispielsweise den CCD-Chip 8 gerichtet werden. Das Umlenkprisma 7 ist dabei derart ausgestaltet, dass eine Strahlumlenkung um einen Winkel von etwa 90°, vorzugsweise von etwas weniger als 90° erzielt wird.

Wie Figur 3 entnommen werden kann, wird also das Bild des illuminierten Zahns 100 mittig durch den hohlzylinderförmigen Vorsprung 6 auf den CCD-Chip 8 zurückgeworfen. Dies bedeutet, dass sich entlang des Handstücks 2 erstreckende Lichtleiter zur Übertragung des Lichts möglichst am Rand verlaufen sollten, um die Bildübertragung nicht zu beeinträchtigen. Um nunmehr eine optimale Lichttransmission zu erzielen, wird eine spezielle Anordnung und Ausrichtung der Lichtquellen und Lichtleiter vorgeschlagen, die nachfolgend anhand der Figuren 3 und 4 erläutert werden soll.

Die Problematik besteht hierbei darin, dass aus Platzgründen zwar die Lichtquellen, die in den Darstellungen mit den Bezugszeichen 9 versehen sind, etwa mittig innerhalb der Griffhülse 3 angeordnet sein sollten, die Lichtleiter selbst allerdings wiederum eher am Rand verlaufen sollten. Gleichzeitig sollten allerdings die Lichtleiter nur bis zu einem bestimmten Radius gebogen werden, um eine optimale Lichttransmission zu gewährleisten. Hierzu wird nunmehr vorgeschlagen, die Lichtquellen 9 im Vergleich zur Längsachse des Handstücks unter einem Winkel von etwa 50-60° anzuordnen. Diese Schrägstellung der Lichtquellen 9 kann insbesondere der Darstellung in Figur 4 entnommen werden, welche die schräge Bestückung der Lichtquellen 9 auf der Platine für die Kameraelektronik zeigt. Diese Schrägstellung hat zur Folge, dass der Biegeradius des sich unmittelbar von den Lichtquellen zur Vorderseite des Handstücks 2 hin erstreckenden Lichtleiters 9a innerhalb eines Bereichs gehalten werden kann, in dem eine maximale Transmission des Lichts gewährleistet ist. Gleichzeitig können die Abmessungen der Griffhülse 3 minimiert werden, so dass ein verhältnismäßig kompaktes und damit gut zu handhabendes Handstück 2 erzielt wird. Alternativ zu der dargestellten Ausführungsform können die Lichtquellen auch in das Gehäuse des Handstücks 2 integriert werden. Sie sind in diesem Fall dann über eine elektrische Leitung mit der Kameraplatine verbunden.

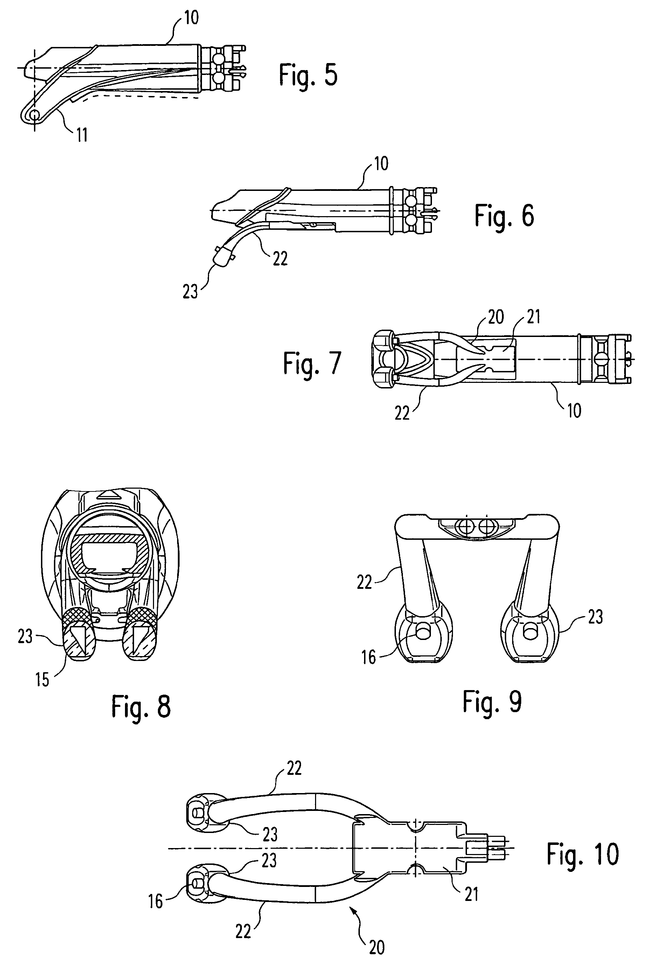

Die Lichtleiter 9a, von denen in den Figur 3 und 4 jeweils einer dargestellt ist, erstrecken sich bis zu einem vorderen Absatz 6a an der Unterseite des Vorsprungs 6. Im Aufgesetzten Zustand des Aufsatzes 10 sind hierbei die vorderen Enden der Lichtleiter 9a auf endseitige Stirnflächen weitere Lichtleiter 9b ausgerichtet, die innerhalb des Aufsatzes 10 bis zu dessen vorderem Ende bis in die Endbereiche der beiden Arme 11 verlaufen. Die Ausgestaltung und Anordnung dieser weiterführenden Lichtleiter 9b soll nachfolgend anhand der Figuren 5-15 näher erläutert werden.

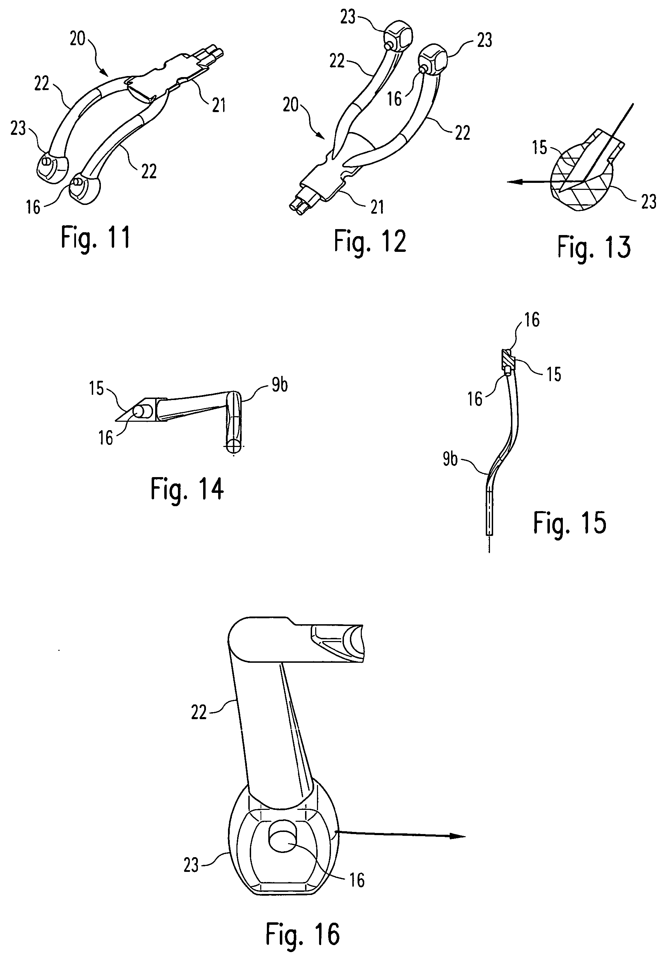

Die Figuren 5 bis 8 zeigen hierbei zunächst verschiedene Ansichten des vorderen Endbereichs des Aufsatzes 10, wobei in den Figuren 6 und 7 auf die Darstellung der äußeren Ummantelung des Aufsatzes 10 verzichtet wurde, so dass der Verlauf der Lichtleiter erkennbar ist. Die Figuren 14 und 15 wiederum zeigen einzelne Lichtleiter 9b, die beispielsweise aus Kunststoff, Glasfaserbündeln ggf. mit teilweise verbackenen Fasern, Glaseinzelfasern, einem Faserstab oder ähnlichen Materialien bestehen und dementsprechend eine gewisse Flexibilität aufweisen. An ihrem vorderen Ende sind die Lichtleiter 9b mit einem Umlenkprisma 15 verbunden, über das - wie später näher erläutert - das Licht in etwa senkrecht umgelenkt und damit auf den zu untersuchenden Zahn gerichtet wird. Die Ankopplung des Lichtleiters 9b an das Umlenkprisma 15 kann beispielsweise durch Anspritzen oder dgl. erfolgen.

Die flexiblen Lichtleiter 9b sind vorzugsweise mit einem optischen Material umspritzt, dessen Brechzahl niedriger ist als die Brechzahl des Kunststofflichtleiters. Durch dieses Material, welches beispielsweise als selbsthaftendes Silikon ausgeführt sein kann, wird die zur optimalen Lichtleitung erforderliche Totalreflexton ermöglicht.

Zur Strahlumlenkung ist an die Austrittsseite des Lichtleiters 9b das bereits erwähnte Umlenkprisma 15 angespritzt, welches zur besseren Transmission zusätzlich noch mit einer Beschichtung auf der Reflexionsfläche 15a versehen sein kann. Die Lichtaustrittsseite 15b wiederum könnte zusätzlich noch mit einer optischen Linsenstruktur versehen werden, wodurch eine Beeinflussung des Lichtstrahls ermöglicht wird.

Einen Besonderheit besteht nunmehr darin, dass die beiden - ggf. ummantelten - Lichtleiter 9b mit den daran befindlichen Prismen 15 in ein entsprechendes Element eingebettet sind, welches nachfolgend als Lichtleiterhalter 20 bezeichnet wird und näher in den Figuren 9-12 dargestellt ist. Dieser Lichtleiterhalter 20 weist zunächst ein Basisteil 21 auf, von dessen Endbereich aus sich die Enden der Lichtleiter 9b erstrecken. Anfangs verlaufen die Lichtleiter 9b parallel in dem Basisteil 21, sie verzweigen dann allerdings in zwei Arme 22 des Lichtleiterhalters 20, die sich zur Vorderseite hin erstrecken. Am vorderen Ende weisen die Arme 22 Kopfbereiche 23 auf, in denen dann jeweils die Umlenkprismen 15 angeordnet sind. An den Umlenkprismen sind hierzu zwei seitlich abstehende Zapfen 16 vorgesehen, über die eine Verankerung der Prismen 15 in Endbereichen 23 des Lichtleiterhalters 20 erzielt wird. Zumindest die vorderen Endbereiche 23 des Lichtleiterhalters 20 bestehen dabei aus einem transparenten bzw. lichtdurchlässigen Material, wobei allerdings die Herstellung des Lichtleiterhalters 20 insgesamt vereinfacht wird, wenn das gesamte Element aus dem gleichen transparenten und flexiblen Material besteht.

Die Einbettung der Lichtleiter 9b in den Lichtleiterhalter 20 hat zur Folge, dass die Lichtleiter 9b besser gegenüber äußeren Einflüssen geschützt sind. Dies ist deshalb von Bedeutung, da der Lichtleiterhalter 20 an dem Aufsatz 10 angeordnet ist, der aus hygienischen Gründen regelmäßig gereinigt, insbesondere allerdings auch sterilisiert werden muss. Dadurch, dass die Lichtleiter 9b und das Umlenkprisma 15 vollständig von dem Lichtleiterhalter 20 ummantelt sind, sind diese optischen Elemente zur Lichtübertragung besser gegenüber den äußeren Einflüssen geschützt. Hierbei kann eine zusätzliche Verbesserung des Schutzes dadurch erzielt werden, dass mit Hilfe einer geeigneten hydrophoben Beschichtung ein Wassereintritt in den Lichtleiter 9b verhindert oder zumindest stark reduziert wird. Der Hintergrund dieser Verbesserung ist, dass beim Autoklavieren der vorzugsweise mit Silikon ummantelte Lichtleiter 9b einer Wasserdampfüberdruck-Atmosphäre ausgesetzt wird. Durch Diffusionsprozesse gelangt das Wasser durch die äußeren Materialschichten in den Kunststofflichtleiter 9b, was zu einer vorzeitigen Alterung bzw. einer Schädigung in Form von Freisetzen von eingefrorenen Spannungen führen kann. Durch Aufbringen einer hydrophoben Schicht, wofür beispielsweise das Material Parylene benutzt werden kann, wird nunmehr verhindert, dass die Feuchtigkeit in die darunterliegenden Bauteile eindringt und diese schädigt. Die hydrophobe Schicht kann hierbei entweder direkt auf den Kunststofflichtleiter oder auf die äußerste Silikonschicht des mit Silikon ummantelten Kunststofflichtleiters aufgebracht werden.

Ein weiterer Vorteil der Einbettung der Lichtleiter 9b sowie der Umlenkprismen 15 in den Lichtleiterhalter 20 besteht ferner darin, dass die Lichteinkopplung in den zu untersuchenden Zahn optimiert werden kann. Wie Figur 16 und insbesondere auch Figur 13, welche eine Schnittdarstellung des vorderen Endbereichs 23 des Lichtleiterhalters 20 mit dem darin eingebetteten Umlenkprisma 15 zeigt, entnommen werden kann, wird das Licht letztendlich über den transparenten Endbereich 23, der nachfolgend auch als Einkopplungskörper bezeichnet wird, in den Zahn eingestrahlt.

Dadurch, dass dieser Einkopplungskörper 23 nunmehr aus einem flexiblen transparenten Material besteht, kann sich dieser möglichst flächig an die Außenseite des Zahns bzw. die Gingiva anlegen. Dieser Kontakt hat zur Folge, dass das Entstehen von Reflektionen oder von Streustrahlung, die auf direktem Weg in das Eintrittsfenster des Aufsatzes 10 eintreten könnten, unterdrückt wird. Störende Reflektionen in dem Bild des illuminierten Zahns können auf diesem Wege reduziert werden. Das flächige Anlegen des Lichteinkopplungskörpers 23 wird dabei vorzugsweise dadurch unterstützt, dass auch die Arme 11 des Aufsatzes 10 aus einem flexiblen Material bestehen und eine gewisse Vorspannung aufweisen, durch welche sich die Arme 11 im auf dem Zahn aufgesetzten Zustand an dessen Seitenwände anschmiegen.

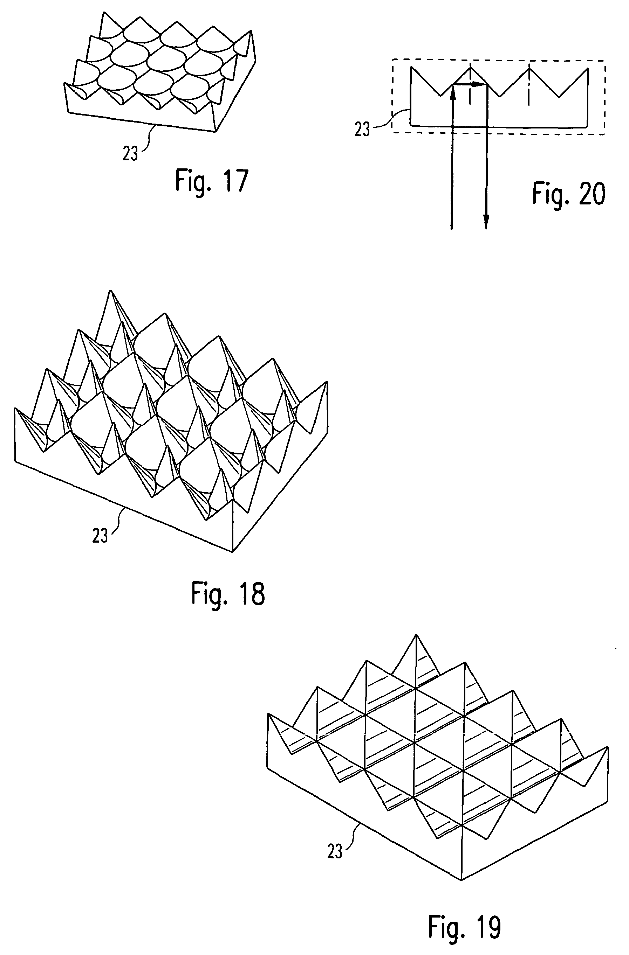

Die Lichteinkopplung in den Zahn kann dabei weiterhin durch eine spezielle Gestaltung der zunächst etwa kugelig ausgestalteten Oberfläche der Einkopplungskörper 23 zusätzlich optimiert werden. Denkbare Oberflächenstrukturen hierfür sind in den Figuren 17-19 dargestellt. Hierbei können Geometrien in Form von Pyramiden, Kegeln, Wellen sowie Wellen mit einer entsprechenden Struktur, die periodisch und/oder unperiodisch verteilt ist, zum Einsatz kommen. Aufgabe dieser Struktur ist es, die Einkopplung des Lichts in die Zahnhartsubstanz und/oder die Gingiva weiter zu verbessern und gleichzeitig zu verhindern, dass Licht, welches nicht eingekoppelt werden kann, an der Zahnhartsubstanz und/oder der Gingiva reflektiert wird, was zu der ungewünschten Überblendung des Bildaufnahmesensors führen würde. Die Funktionsweise dieser Struktur beruht hierbei darauf, dass Lichtstrahlen an den Flächen der Strukturen teilweise umgelenkt werden, solange diese Strukturen nicht mit der Zahnhartsubstanz und/oder Gingiva in Kontakt kommen. Dieser Effekt beruht auf dem Unterschied im Brechungsindex zwischen dem Kontaktmaterial und Luft. Aus diesem Unterschied ergibt sich ein entsprechender Winkel der Totalreflektion, wobei Lichtstrahlen, die unter einem kleineren Winkel auf die Oberfläche der Struktur auftreffen, zurückreflektiert werden, wie dies in Figur 20 schematisch dargestellt ist. Kommt nunmehr allerdings die Struktur mit der Zahnhartsubstanz und/oder der Gingiva in Kontakt, so werden die geometrisch bestimmten Lichtaustrittsflächen deformiert, was eine Veränderung des Winkels, unter dem der Lichtstrahl auf die Grenzfläche auftrifft zur Folge hat. Dies wiederum ermöglicht nunmehr, dass die Lichtstrahlen den Lichteinkopplungskörper 23 verlassen und in die Zahnhartsubstanz eintreten können.

Eine weitere Möglichkeit zur Vermeidung von Überblendungen würde in der Verwendung von Polfiltern auf der Lichtaustrittsseite sowie auf der Lichteintrittsseite und/oder in dem optischen System des Detektionsgeräts bestehen. Die Filter sind dabei derart ausgerichtet, dass eine direkte Einstrahlung des Lichtaustrittsendes zur Lichteintrittsseite verhindert wird.

Schließlich besteht ein Vorteil der Einbettung der Lichtleiter 9b mit den Umlenkprismen 16 in den Lichtleiterhalter 20 auch darin, dass der Lichtleiterhalter 20 in einfacher Weise ausgetauscht werden kann, sollte dies aufgrund von Alterungserscheinungen erforderlich sein. Es ist in diesem Fall nicht erforderlich, den kompletten Aufsatz 10 auszuwechseln, weshalb der Kostenaufwand für entsprechende Wartungsarbeiten reduziert werden kann.

Die bislang beschriebenen Maßnahmen tragen insbesondere dazu bei, dass eine verbesserte Lichteinkopplung in den zu untersuchenden Zahn erzielt werden kann. Eine weitere Verbesserung des Systems kann ferner auch darin bestehen, Licht mit einer verhältnismäßig geringen spektralen Bandbreite im Bereich zwischen 2 und 10 nm zu verwenden. Dies bringt insbesondere den Vorteil mit sich, dass sogenannte Speckle, die durch Interferenzerscheinungen im humanen Gewebe hervorgerufen werden, vermieden oder zumindest deutlich reduziert werden können. Auch dies trägt zu einer Optimierung der Untersuchungsergebnisse bei.

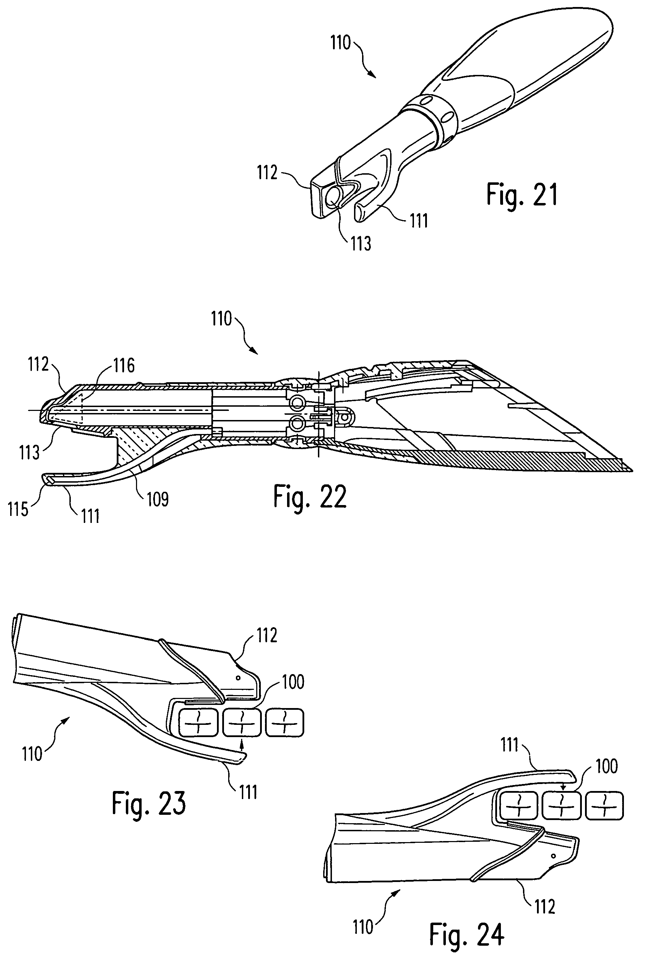

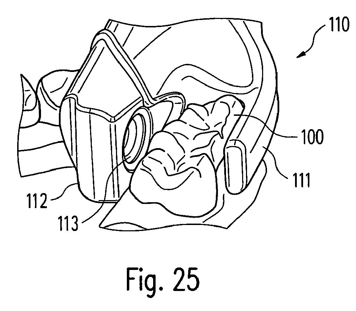

Die Figuren 21-25 zeigen schließlich einen weiteren Aufsatz 110, der bei dem erfindungsgemäßen Gerät zur Kariesdiagnose zum Einsatz kommen könnte. Im Unterschied zu dem zuvor diskutierten Aufsatz 10 weist dieser weitere Aufsatz 110 lediglich einen einzigen Arm 111 auf, durch den sich ein Lichtleiter 109 mit einem am vorderen Ende befindlichen Umlenkelement 115 erstreckt. Diesem vorderen Ende des Arms 111 gegenüberliegend ist wiederum der Endbereich 112 mit einem Lichteintrittsfenster 113 angeordnet. Im aufgesetzten Zustand des Aufsatzes 110 auf der Griffhülse 3 des Handstücks 2 befindet sich unmittelbar hinter dem Eintrittsfenster 113 das Umlenkprisma 116, mit dessen Hilfe das von dem zu untersuchenden Zahn 100 einfallende Licht auf die Bilderfassungsmittel gerichtet wird.

Die einander gegenüberliegende Anordnung von Umlenkelement 115 und Lichteintrittsfenster 113 hat zur Folge, dass mit Hilfe dieses Aufsatzes 110 der zu untersuchende Zahn 100 wahlweise von der sogenannten bukalen Zahnfläche beleuchtet und der lingualen Zahnfläche beobachtet werden kann oder alternativ hierzu auch von der lingualen Zahnfläche beleuchtet und von der bukalen Zahnfläche aus beobachtet werden kann. Im Vergleich zu bislang üblichen Ausführungsformen ist bei einem derartigen Wechsel kein Austausch des Aufsatzes erforderlich, was eine wesentliche Arbeitserleichterung und Zeitersparnis darstellt. Ferner muss das Handstück auch nicht aus dem Arbeitsraum, also dem Mundraum des Patienten herausgenommen werden und zum Wechsel der Betrachtungsrichtung ist keine Zweihandbedienung erforderlich. Der Aufsatz 110 ist hierbei derart auf dem Handstück 2 angeordnet, dass er nicht verdreht werden kann. Hierdurch wird eine ergonomische Handhabung erzielt.

Ferner kann auch bei diesem Ausführungsbeispiel des Aufsatzes 110 der Lichtleiter 109 mit dem Umlenkelement 115 in ein transparentes flexibles Element eingebettet sein. Das heißt, die zuvor beschriebenen Maßnahmen zur Verbesserung der Lichteinkopplung in den Zahn können auch hier zum Einsatz kommen.

Somit wird insgesamt ein zahnärztliches System zum Transilluminieren von Zähnen geschaffen, welches die Erstellung qualitativ hochwertiger Bildaufnahmen ermöglicht, was letztendlich zu einer deutlichen Verbesserung der Karieserkennung führt.

## Patentansprüche

1. Zahnärztliches System zum Transilluminieren von Zähnen, aufweisend:
eine Lichtquelle (9) zum Erzeugen einer Untersuchungsstrahlung,
Bestrahlungsmittel zum Richten der Untersuchungsstrahlung auf einen zu untersuchenden Zahn (100), sowie
Mittel zum Erfassen eines optischen Bildes des durch die Untersuchungsstrahlung illuminierten Zahns (100),
wobei die Bestrahlungsmittel lichtleitende und/oder lichtlenkende Elemente umfassen,
**dadurch gekennzeichnet,**
**dass** zumindest das dem zu untersuchenden Zahn zugewandte lichtleitende und/oder lichtlenkende Element in einen transparenten, flexiblen Einkopplungskörper (23) eingebettet ist, der zur Anlage an den Zahn (100) bzw. die Gingiva des Zahns (100) vorgesehen ist,
wobei der Einkopplungskörper (23) in seinem zur Lichteinkopplung vorgesehenen Bereich eine Oberfläche aufweist, die die Form einer Kugel oder eines Kugelsegments aufweist oder konvex oder konkav ausgebildet ist,
wobei der Einkopplungskörper (23) in seinem zur Lichteinkopplung vorgesehenen Bereich mit einer Zusatzstruktur versehen ist,
wobei durch die Zusatzstruktur deformierbare Lichtaustrittsflächen gebildet sind.

2. Zahnärztliches System nach einem der vorherigen Ansprüche,
**dadurch gekennzeichnet,**
**dass** die Bestrahlungsmittel zumindest einen Lichtleiter (9b) umfassen, der in das Material des Einkopplungskörpers (23) eingebettet ist.

3. Zahnärztliches System nach Anspruch 2,
**dadurch gekennzeichnet,**
**dass** der Einkopplungskörper (23) Bestandteil eines Lichtleiterhalters (20) ist, in den der Lichtleiter (9b) eingebettet ist.

4. Zahnärztliches System nach Anspruch 2 oder 3,
**dadurch gekennzeichnet,**
**dass** am lichtausgangsseitigen Ende des Lichtleiters (9b) ein Umlenkprisma (15) angeordnet ist, welches in den Einkopplungskörper (23) eingebettet ist.

5. Zahnärztliches System nach einem der Ansprüche 2 bis 4,
**dadurch gekennzeichnet,**
**dass** der Lichtleiter (9b) und/oder eine Ummantelung des Lichtleiters (9b) eine hydrophobe Beschichtung aufweist.

6. Zahnärztliches System nach einem der vorherigen Ansprüche,
**dadurch gekennzeichnet,**
**dass** die Lichtquelle (9) dazu ausgebildet ist, Licht mit einer spektralen Bandweite im Bereich von 2 bis 10 nm zu emittieren.

7. Zahnärztliches System nach einem der vorherigen Ansprüche,
**dadurch gekennzeichnet,**
**dass** dieses ein Handstück (2) aufweist, in dem die Lichtquelle (9) angeordnet ist, wobei die Lichtquelle (9) gegenüber der Handstücklängsachse geneigt angeordnet ist.

8. Zahnärztliches System nach einem der vorherigen Ansprüche,
**dadurch gekennzeichnet,**
**dass** dieses ein Handstück (2) aufweist und zumindest ein Teil der Bestrahlungsmittel in einem abnehmbaren Aufsatz (110) angeordnet sind, wobei der Aufsatz (110) gegenüber der Handstücklängsachse nicht verdrehbar ist.

## Claims

1. A dental system (1) for the transillumination of teeth, having:
a light source (9) for producing examination radiation,
irradiation means for directing the examination radiation onto a tooth (100) to be examined, and also means for acquiring an optical image of the tooth (100) illuminated by the examination radiation, wherein the irradiation means include light-guiding and/or light-deflecting elements,
**characterised in that**
at least the light-guiding and/or light-deflecting element facing the tooth to be examined is embedded in a transparent, flexible coupling body (23) which is provided to rest against the tooth (100) or the gingiva of the tooth (100),
wherein the coupling body (23), in its region provided for light-coupling, has a surface which has the form of a sphere or a spherical segment or is configured so as to be convex or concave,
wherein the coupling body (23) is provided with an additional structure in its region provided for light-coupling,
wherein light-emitting faces are constituted that can be deformed by the additional structure.

2. A dental system according to one of the preceding claims,
**characterised in that**
the irradiation means include at least one light guide (9b) which is embedded in the material of the coupling body (23).

3. A dental system according to claim 2,
**characterised in that**
the coupling body (23) is part of a light-guide holder (20) in which the light guide (9b) is embedded.

4. A dental system according to claim 2 or 3,
**characterised in that**
arranged at the end of the light guide (9b) on the light-exit side there is a deflecting prism (15),
which is embedded in the coupling body (23).

5. A dental system according to one of claims 2 to 4,
**characterised in that**
the light guide (9b) and/or a sheathing of the light guide (9b) have/has a hydrophobic coating.

6. A dental system according to one of the preceding claims,
**characterised in that**
the light source (9) is configured to emit light having a spectral bandwidth in the range from 2 to 10 nm.

7. A dental system according to one of the preceding claims,
**characterised in that**
it has a handpiece (2) in which the light source (9) is arranged, wherein the light source (9) is arranged so as to be inclined with respect to the longitudinal axis of the handpiece.

8. A dental system according to one of the preceding claims,
**characterised in that**
it has a handpiece (2), and at least part of the irradiation means is arranged in a removable attachment (110), wherein the attachment (110) cannot be twisted with respect to the longitudinal axis of the handpiece.

## Revendications

1. Système de médecine dentaire pour la transillumination de dents, présentant :
une source de lumière (9) pour générer un rayonnement d'examen,
des moyens de rayonnement pour diriger le rayon d'examen sur une dent à examiner (100), ainsi que
des moyens de saisie d'une image optique de la dent illuminée par le rayonnement d'examen (100),
dans lequel les moyens de rayonnement comprennent des éléments conduisant la lumière et/ou déviant la lumière,
**caractérisé en ce que**,
au moins l'élément conduisant la lumière et/ou déviant la lumière tourné vers la dent à examiner est englobé dans un corps de couplage (23) transparent, flexible, qui est prévu pour être appliqué sur la dent (100) ou la gencive de la dent (100),
dans lequel le corps de couplage (23) présente dans sa zone prévue pour le couplage lumineux, une surface qui présente la forme d'une sphère ou d'un segment sphérique ou est conçue de façon convexe ou concave,
dans lequel le corps de couplage (23) est doté d'une structure supplémentaire dans sa zone prévue pour le couplage lumineux,
dans lequel des surfaces de sortie de lumière déformables sont formées par la structure supplémentaire.

2. Système de médecine dentaire selon l'une des revendications précédentes,
**caractérisé en ce que**
les moyens de rayonnement comprennent au moins un guide de lumière (9b) qui est englobé dans le matériau du corps de couplage (23).

3. Système de médecine dentaire selon la revendication 2,
**caractérisé en ce que**
le corps de couplage (23) fait partie d'un support de guide de lumière (20) dans lequel le guide de lumière (9b) est englobé.

4. Système de médecine dentaire selon la revendication 2 ou 3,
**caractérisé en ce que**
à l'extrémité du côté de sortie de la lumière du guide de lumière (9b) est disposé un prisme déflecteur (15) qui est englobé dans le corps de couplage (23).

5. Système de médecine dentaire selon l'une des revendications 2 à 4,
**caractérisé en ce que**
le guide de lumière (9b) et/ou une gaine du guide de lumière (9b) présente un revêtement hydrophobe.

6. Système de médecine dentaire selon l'une des revendications précédentes,
**caractérisé en ce que**
la source de lumière (9) est conçue de façon à émettre de la lumière ayant un spectre de bande passante dans la plage de 2 à 10 nm.

7. Système de médecine dentaire selon l'une des revendications précédentes,
**caractérisé en ce que**
celui-ci présente une pièce à main (2) dans laquelle la source de lumière (9) est disposée, dans lequel la source de lumière (9) est disposée de façon inclinée par rapport à l'axe longitudinal de la pièce à main.

8. Système de médecine dentaire selon l'une des revendications précédentes,
**caractérisé en ce que**
celui-ci présente une pièce à main (2) et au moins une partie des moyens de rayonnement sont disposés dans un accessoire amovible (110), dans lequel l'accessoire (110) ne peut pas tourner par rapport à l'axe longitudinal de la pièce à main.
